# EUROPEAN PATENT APPLICATION

(11) **EP 2 777 733 A2**
(43) Date of publication of application: **17.09.2014**
(21) Application number: 14159455.6
(22) Date of filing: 13.03.2014
(51) Int. Cl.: A61M 13/00

(54) **Gas regulator, control interface module, and methods for surgical applications**

(30) Priority: 14.03.2013 US 201361783773 P; 10.05.2013 US 201361821993 P
(71) Applicant: Nordson Corporation, Westlake, Ohio 44145 (US)
(72) Inventor: Hoogenakker, Jon E, Minnesota, Minnesota 55077 (US); Lou, Huadong, Lauderdale, Minnesota 55108 (US); Robb, Bradley D, Maple Plain, Minnesota 55359 (US)
(74) Representative: Findlay, Alice Rosemary

(57) **Abstract**

A regulator for regulating the pressure and flow of gas traveling between a gas inlet and a gas outlet. A first pressure regulator in fluid communication with the gas inlet and configured to prevent gas flowing downstream of the first pressure regulator from exceeding a first pressure. A first line between the first pressure regulator and the gas outlet. A second pressure reducing device in fluid communication with the first line downstream of the first pressure regulator and configured to prevent gas flowing in the first line at a point downstream of the second pressure regulator from exceeding a second pressure. A control valve actuatable to an open position when gas is transmitted to a first pilot valve at or below the second pressure, the first pilot valve in fluid communication with the first line.

## Description

The invention generally relates to the use of pressurized gas in various surgical applications, and more specifically to gas-assisted delivery of biomaterials.

During laparoscopic procedures, it is common practice to insufflate the abdominal cavity, or create pneumoperitoneum, by introducing a gas into the abdominal cavity with an insufflator. Insufflation thereby expands the abdominal cavity to aid in viewing, providing access to, and manipulating certain internal structures. Once the abdominal cavity is insufflated, a series of incisions are created in the abdominal wall and a trocar is inserted into each of the incisions. The trocars include a self-closing seal for preventing loss of insufflation gas while allowing penetration by the surgical instruments employed. More particularly, one of the trocars is meant to accommodate a camera and the other(s) are meant to accommodate a working instrument, such as the device disclosed in U.S. Patent No. 8,603,025, issued December 10, 2013, and entitled GAS-ASSISTED FLUID-DISPENSING DEVICE.

Practitioners aim to maintain a relatively constant insufflation pressure of approximately 15 mmHg. Maintaining the insufflation pressure is often a challenge, particularly during certain procedures where additional gas is introduced from the working instrument into the abdominal cavity. The additional gas introduced by the instrument increases the pressure within the abdomen. Moreover, as a result of certain procedures such as electrocautery, smoke and/or other particulates may build up within the abdominal cavity, thus obstructing the camera and therefore the practitioners' view of the abdominal cavity. While some trocars and insufflators in the prior art include passive vents to counteract increases in insufflation pressure, these vents are insufficient to quickly ameliorate the potentially high pressures that may occur within the abdomen. Moreover, such passive vents may be slow when it comes to clearing smoke and particulates from the abdominal cavity. As a result, practitioners are required to wait longer periods of time for the abdomen to clear of smoke and other particulates to resume the procedure. Furthermore, a slow rate of venting may lead to the particulates settling within the tissue in an undesired manner, rather than leaving the abdominal cavity through the vent.

The introduction of additional gas by a laparoscopic working instrument could result in over-inflation and distention of the abdominal wall. Moreover, most laparoscopic spray applicators in the art apply gas to the abdominal cavity between approximately 27 and 30 psi. Such high pressures are often required due to the pressure requirements associated with opening and closing certain pneumatic valves. Such high pressures create unwanted danger to the patient, such as distention of the abdominal wall and gas embolism. While it is desirable to reduce the application pressure, current instruments in the art are incapable and/or ineffective operating at these lower pressures.

Moreover, in some situations, more than one gas regulator may be needed in the operating theatre, depending on the procedure and the preferences of the practitioner. A gas regulator is a device that fluidly communicates or connects the gas input from the operating theatre and a working instrument that employs gas pressure to, for example, dispense certain materials (such as the gas-assisted device or applicator mentioned above). Gas regulators typically employ a somewhat intricate series of fluid lines, valves, and flow- and/or pressure-controlling devices for regulating the characteristics of the gas that is dispensed from the working instrument. Most of the components of gas regulators are typically housed within a control interface module, which may include external switches, knobs, and other mechanisms for controlling the settings and/or operation of the gas regulator. Because of the different pressure and flow characteristics associated with different procedures, one gas regulator is employed when a topical dispensing device, for example, is used, and another, different gas regulator is employed when a laparoscopic dispensing device, for example, is used. Moreover, some procedures require multiple inputs of gas, often at different characteristics (i.e., pressure and flow). In either of these situations, the current state of the art calls for two separate control interface modules having separate regulators in the operating theatre. Thus, valuable and limited space in the operating theatre is often wasted.

In one embodiment of the invention, a control interface module is provided and is configured to control an internal regulator for regulating a pressure and flow of a pressurized gas flowing from a gas inlet to first and second gas outlets. The control interface module comprises a housing having a gas inlet and first and second gas outlets. The control interface module further comprises a multiple position switch on the housing. The switch is configured to control whether the gas is to permitted to flow from only one of, each of, or neither of the first and second outlets.

A method of regulating a pressure and flow of a pressurized gas traveling between a gas inlet and a gas outlet of a regulator is provided and comprises introducing pressurized gas into a first line of the regulator from an inlet of the regulator. The method further comprises reducing the pressure of the gas to a first pressure using a first pressure regulator, thereby preventing gas flowing downstream of the first pressure regulator in the first line from exceeding the first pressure. The method further comprises reducing the pressure of the gas flowing in the first line to a second pressure using a second pressure regulator, thereby preventing gas flowing downstream of the second pressure regulator in the first line from exceeding the second pressure. The method further comprises transmitting the pressurized gas at or below the second pressure to a first pilot valve, thereby actuating a control valve to an open position. The method further comprises transmitting the gas at or below the first pressure to a second pilot valve, thereby actuating a pneumatic valve to an open position, and permitting the pressurized gas to flow downstream of the pneumatic valve in the first line and thereby flow out of the gas outlet when the pneumatic valve is in the open position. In another embodiment, a method of regulating a pressure of gas traveling between a gas inlet and a gas outlet is provided. The gas inlet is adapted for connection to a source of gas under pressure. The method comprises reducing the pressure of the gas with a first pressure regulator to a first pressure, thereby preventing gas flowing downstream of the first pressure regulator in a first line from exceeding the first pressure. The method further comprises reducing the pressure of the gas with a second pressure regulator to a second pressure, thereby preventing gas flowing downstream of the second pressure regulator in the first line from exceeding the second pressure. The method further comprises transmitting gas at or below the second pressure to a first pilot valve, thereby actuating a control valve to an open position thereof. As the control valve actuates to an open position, gas is transmitted to a second pilot valve at or below the first pressure, thereby actuating a pneumatic valve to an open position thereof. The gas in the first line is permitted to exit from the gas outlet when the pneumatic valve is in the open position.

In another embodiment, a regulator for use with a gas-assisted applicator in an endoscopic or a laparoscopic procedure whereby the device is in fluid communication with a body cavity is also provided. The regulator comprises an inlet configured to be fluidly communicated with a source of pressurized gas, and an outlet in fluid communication with the inlet. The outlet is configured to be coupled with an applicator. The regulator further comprises a vent system including a vent line leading to a vent outlet, and a vacuum extraction system in fluid communication with the vent line. The vent line is configured to be fluidly communicated with the body cavity such that gas within the body cavity may be drawn into the vent line and out of the vent outlet during operation of the vacuum extraction system.

In another embodiment, a method of venting a body cavity during a laparoscopic or endoscopic procedure using a regulator is provided. The regulator comprises an inlet configured to be fluidly communicated with a source of pressurized gas and an outlet configured to be coupled with a gas-assisted endoscopic or laparoscopic applicator. The outlet is in fluid communication with the inlet such that gas flowing into the inlet may flow to and be dispensed by the device into the body cavity. The regulator further comprises a vent system including a vent line leading to a vent outlet, and a vacuum extraction system in fluid communication with the vent line. The method comprises fluidly communicating the inlet to a source of pressurized gas, fluidly communicating the vent line to the body cavity, and operating the vacuum extraction system such that gas within the body cavity may be drawn through the vent line and out of the vent outlet.

A method of performing a gas-assisted endoscopic or laparoscopic procedure using a gas-assisted applicator and a regulator is provided. The regulator comprises an inlet, an outlet fluidly communicating with the inlet such that gas flowing into the inlet may flow to the outlet, and a vent system including a vent line leading to a vent outlet. The regulator further comprises and a vacuum extraction system in fluid communication with the vent line. The method comprises fluidly communicating the inlet to a source of pressurized gas, fluidly communicating the vent line with the body cavity, and fluidly communicating the gas-assisted applicator with the outlet. The method further comprises directing the gas-assisted applicator into the body cavity and directing gas into the body cavity from the gas-assisted applicator. The method further comprises operating the vacuum extraction system such that gas within the body cavity may be drawn through the vent line and out of the vent outlet.

The invention will now be further described by way of example with reference to the accompanying drawings, in which:
Fig. 1 is a perspective view of a control interface module according to one embodiment.
Fig. 2 is a perspective view of a control interface module according to an alternative embodiment.
Fig. 3 is a perspective view of a control interface module according to another alternative embodiment.
Fig. 4 is a perspective view of a control interface module according to another alternative embodiment.
Fig. 5 is a pneumatic schematic diagram of a regulator according to an alternative embodiment.
Fig. 6A is a pneumatic schematic diagram of a regulator according to another alternative embodiment.
Fig. 6B is a detailed side view of a plunger link switch assembly.
Fig. 6C is a detailed front view of the plunger link switch assembly of Fig. 6B.
Fig. 7 is a pneumatic schematic diagram according to yet another alternative embodiment.
Fig. 8 is a pneumatic schematic diagram of a regulator according to yet another alternative embodiment.
Fig. 9 is a pneumatic schematic diagram of a regulator according to yet another alternative embodiment.
Fig. 10 is a pneumatic schematic diagram of a regulator according to yet another alternative embodiment.
Fig. 11 is a perspective view of a control interface module according to another alternative embodiment.
Fig. 12 is a pneumatic schematic diagram of a regulator according to yet another alternative embodiment.
Fig. 13 is a perspective view of a control interface module according to another alternative embodiment.
Figs. 14A through 14C are schematic views showing various manners of operation of a control interface module.

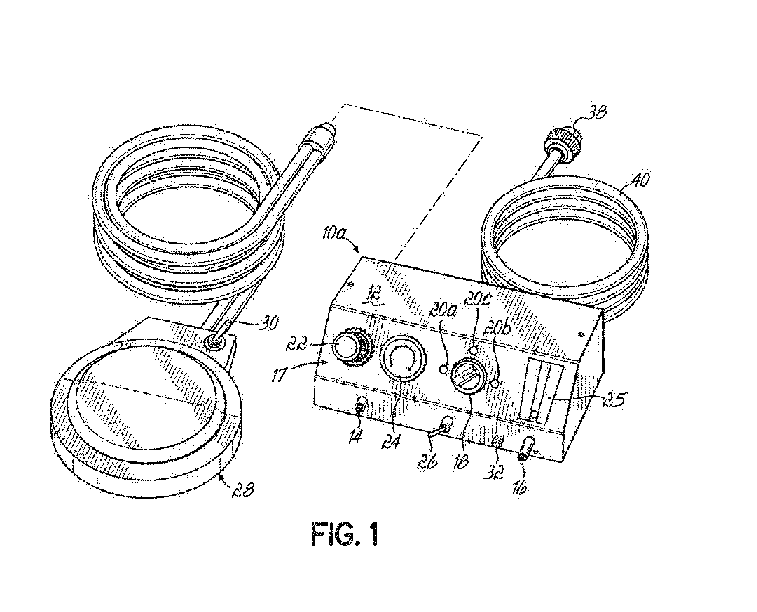

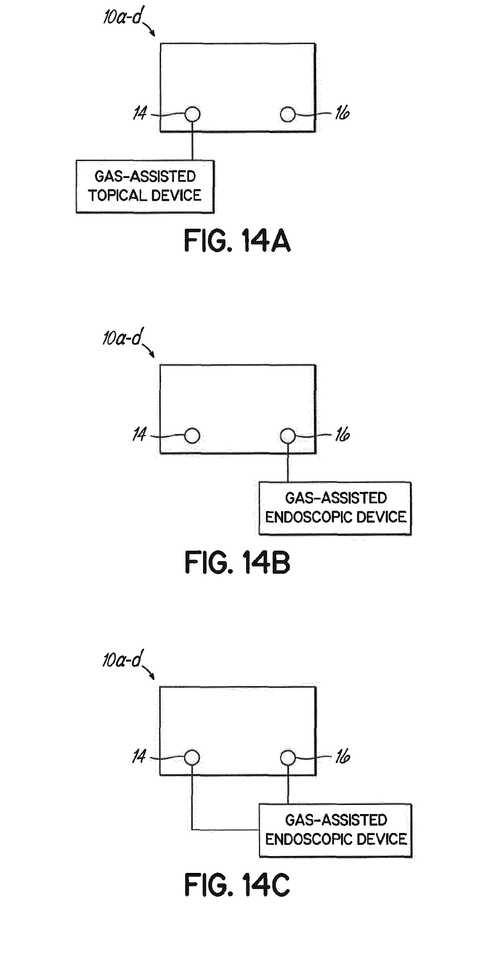

Referring to Fig. 1, one embodiment of a control interface module 10a is shown. The control interface module 10a is configured to control a gas regulator, such as one of those described herein, most of the components of which are contained inside of the housing 12. The gas regulator includes an input port (not shown) to receive a gas input from gas source (not shown) via hose member 13 and is operable to regulate the characteristics of the gas that is dispensed from one or both of the first or second outlet ports 14, 16 of the module 10a. Particularly, the regulator is configured to regulate the characteristics of the gas, such as pressure and flow, dispensed from one of both of the first or second outlet ports 14, 16 such that the control interface module 10a may be utilized for multiple types of procedures, as will be described in more detail below (and examples of which can be seen in Figs. 14A-C). In that regard, housing 12 includes a control panel 17 a multiple position switch 18 having first, second or third positions which indicate whether the regulator is in a first, second, or third mode. The first and second modes may, for example, be associated with different types of procedures. In the third mode, gas may not flow from either of the first or second outlet ports 14, 16. In other words, in the third mode, the regulator does not operate.

In the first mode, gas may continuously flow from the first outlet port 14 at a rate of approximately 2.5 liters per minute (LPM). The flow rate from the first outlet port 14 may be pre-set at the factory or in the operating environment. While gas flows from the first outlet port 14 at a rate of approximately 2.5 LPM, the preset flow rate in other embodiments may be between approximately 0 LPM and approximately 15 LPM, depending on the particular application. The pressure of the gas flowing from the first outlet port 14 may be adjusted by the rotatable knob 22. A pressure gauge 24 on the control interface module 10a provides a visual indication of the pressure of the gas flowing through the first outlet port 14 (in any of the first, second, or third modes). In one embodiment, the pressure of the gas flowing through the first outlet port 14 is adjustable between approximately 0 psi and approximately 15 psi. The first mode, in at least one embodiment, may be utilized when a topical applicator or device is connected to the first outlet port 14. More specifically, the gas from the first outlet port 14 may be used in the gas assisted dispensing of one or more materials, in the form of a liquid or powder, for example, from a dispensing device, such as the one described in US 8,603,025. In that regard, the one or more materials may be advanced within the device (i.e., mechanically or another manner) to a tip or other portion whereby the pressure of the gas from first outlet port 14 assists in dispensing the material from the tip. Therefore, in some instances, the gas may flow from the first outlet port 14 and thus the device without dispensing any of the material, depending on the position of the one or more materials relative to the tip. Essentially, gas flowing from the first outlet port 14 then flows into tubing to the tip, whereby the gas may interact with the materials delivered to the tip of the applicator. (i.e., Figs. 14A or 14B). Thus, the gas from the first outlet port 14 assists in dispensing the materials to a topical treatment site. However, the first mode is not limited to such an application and it is anticipated that the first mode is applicable to a wide range of applications.

In the second mode, gas may flow from each of the first and second outlet ports 14, 16. As in the first mode, the gas flows from the first outlet port 14 at approximately 2.5 LPM. Similarly, the pressure of the gas exiting the first outlet port 14 may adjustable by the knob 22 between approximately 0 psi and approximately 15 psi. In the second mode, gas may flow from the second outlet port 16 at a preset flow rate which, in one embodiment, is between 0.5 LPM and approximately 4 LPM. The flow rate of the gas flowing from the second outlet port 16 may be indicated on flow meter 25. The first and second outlet ports 14, 16 may be fluidly connected to a gas-assisted fluid-dispensing device, whereby the gas from each of the first and second outlet ports 14, 16 may be utilized for different purposes that would otherwise require separate regulators and thus separate control interface modules.

In that regard, first outlet port 14 may be fluidly connected to a portion of the dispensing device (such as a plunger, e.g.) in order to deliver, essentially in a pneumatically assisted manner, the one or more materials to the tip of the device. Utilizing the gas pressure from first outlet port 14 to pneumatically advance the one or more materials to the tip is particularly advantageous in endoscopic or laparoscopic applications where utilizing solely a mechanical plunger would be difficult or infeasible. On the other hand, the second outlet port 16 may be in fluid communication with a portion of the device, such as tubing leading to a point adjacent the tip of the device. Gas flowing from the second outlet port 16 then flows into tubing and then to the tip, whereby the gas may interact with the one or more materials delivered to the tip of the device. Thus, the gas from the second outlet port 16 may effectively assist in dispensing the materials to a treatment site (i.e., Fig. 14C). In the third mode, gas is not permitted to flow through either of the first or second outlet ports 14, 16.

The control interface module 10a includes further control mechanisms associated with the operating modes. More particularly, module 10a includes a toggle switch 26 having on and off positions. When the toggle switch 26 is in the on position, and the regulator is in the second mode, gas continuously flows to the tip of the device that is fluidly connected to the second outlet port 16. The continuous flow of gas to the tip of the device prevents the condensation from forming on the tip of the device, which may block the device from delivering the one or more materials. Moreover, the continuous flow of gas to the device (i.e. tubing, as described above) clears the tip of materials, such as blood, in the event that the tip comes into contact with bodily tissues.

Control interface module 10a further includes a foot switch 28 which is operable to control the flow of gas from the first and second outlet ports 14, 16, when the regulator is in the second mode. More particularly, foot switch 28 includes a secondary toggle switch 30 having on and off positions. When the secondary toggle switch 30 is in the on position, gas may flow continuously to the first and second outlets 14, 16 (provided that the regulator is in the second mode). On the other hand, when the secondary toggle switch 30 is in the off position, gas may only flow through the first and second outlet ports 14, 16 upon the depression of the foot switch 26 (provided that the regulator is in the second mode).

When the secondary toggle switch 30 is in the on position and the foot switch 26 is not depressed, the flow rate of the continuous flow of gas from the second outlet port 16 is between approximately 0 LPM and approximately 1 LPM (provided that the regulator is in the second mode). However, where increased flow is desired, the depression of foot switch 28 (when the secondary toggle switch 30 is in the on position) may increase the flow rate of gas flowing out of second outlet port 16 to a rate of between approximately 1 LPM and approximately 4 LPM (provided that the regulator is in the second mode).

In the embodiment shown, the pressure of the gas flowing out of the second outlet port 16 is constant such that it is not adjustable. In one embodiment, the pressure of the gas flowing from the second outlet port 16 is between approximately 0 psi and approximately 1 psi.

As disclosed above, the second mode may be used in applications where the one or more materials are delivered endoscopically or laparoscopically into the body cavity. In those applications, a vent line of the regulator in fluid communication with vent input port 32 may be used for purposes of counteracting the increased volume and/or pressure of gas inside the abdominal or other body cavity when biomaterials are directed into a body cavity of the body under gas pressure, as described hereinabove. More specifically, vent input port 32 operates, or opens, when gas is flowing through the second outlet port 16.

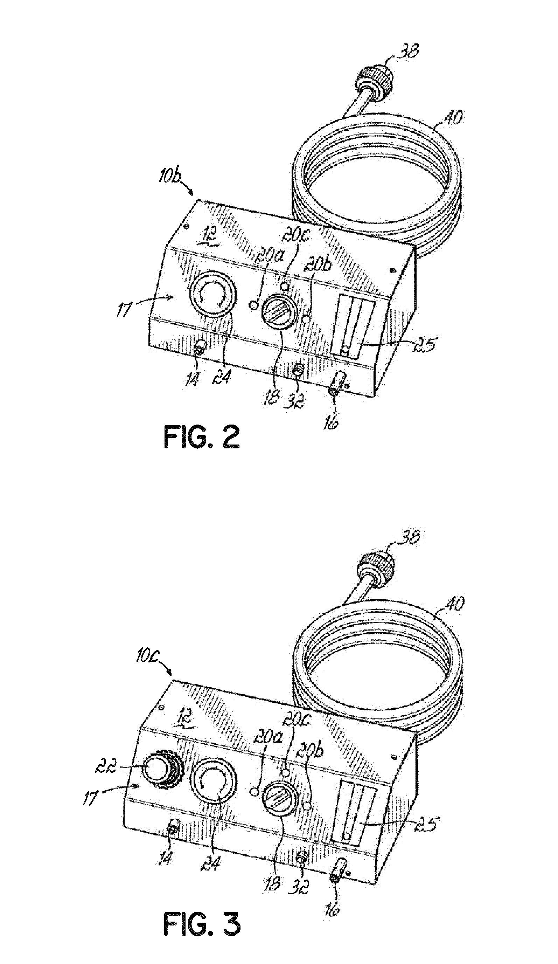

Referring to Fig. 2, an alternative embodiment of a control interface module 10b for controlling a regulator (not shown) is shown in Fig. 2. Many of the components the embodiment shown in Fig. 2 of the control interface module 10b are identical or substantially similar to the components described above with reference to the embodiment shown in Fig. 1, and these components have been marked with the same reference numbers without additional explanation below. Functionally, control interface module 10b is very similar to the embodiment of module 10a shown Fig. 1. In that regard, module 10b may be used for controlling the regulator such that it operates in a first mode, whereby the first outlet port 14 may be fluidly communicated with a topical fluid-assisted dispensing device, and in a second mode, whereby first and second outlet ports 14, 16 may be fluidly connected with a laparoscopic or endoscopic fluid-assisted dispensing device (as described above). When the regulator is in the first mode, gas may continuously flow from the first outlet port 14 in a substantially similar manner as described with respect to the embodiment of Fig. 1. More particularly, gas may continuously flow from the first outlet port 14 at a rate of approximately 2.5 LPM. The flow rate of gas flowing from the first outlet port 14 may be pre-set at the factory or may be adjusted or set in the operating environment. While gas flows from the first outlet port 14 at a rate of approximately 2.5 LPM, the preset flow rate in other embodiments may be between approximately 0 LPM and approximately 15 LPM, depending on the particular application. Unlike the embodiment shown in Fig. 1, however, the module 10b of Fig. 1 does not include a knob for adjusting pressure. Thus, the pressure of gas flowing from the first outlet port 14 may be set at a substantially constant value the factory or in the operating environment. Preferably, the pressure of gas flowing from the first outlet (in the first and second modes) is between approximately 0 psi and approximately 15 psi.

When the regulator is in the second mode, gas may flow from the each of the first and second outlet ports 14, 16. In the second mode, gas may flow from the first outlet port 14 in the same manner as described with respect to the first mode. Gas may flow from the second outlet port 16 with the same or substantially similar properties as described above with respect to the embodiment described and shown in Fig. 1. Optionally, module 10b may include a foot switch 28, which is operable to control the flow of gas from the first and second outlet ports 14, 16, when the regulator is in the second mode. More particularly, foot switch 28 includes a toggle switch 30 having on and off positions. When the secondary toggle switch 30 is in the on position, gas may flow continuously to the first and second outlet ports 14, 16 (provided that the regulator is in the second mode). On the other hand, when the secondary toggle switch 30 is in the off position, gas may only flow through the first and second outlet ports 14, 16 upon the depression of the foot switch 26 (provided that the regulator is in the second mode). In embodiments where the module 10b is not provided with a foot switch 28, gas may flow from each of the first and second outlet ports 14, 16 upon switch 18 being moved to the position associated with the second mode.

Another alternative embodiment of a control interface module 10c for controlling a regulator (not shown) is shown in Fig. 3. Many of the components the embodiment shown in Fig. 3 of the control interface module 10c are identical or substantially similar to the components described above with reference to the embodiment shown in Figs. 1 and 2, and these components have been marked with the same reference numbers without additional explanation below. Functionally, control interface module 10c is very similar to the embodiment of module 10 shown Fig. 1 and module 10b shown in Fig. 2. In that regard, module 10c may be used for controlling operating modes the regulator. Regulator associated with control interface module 10c includes first, second and third operating modes. However, unlike the embodiments shown in Figs. 1 and 2, the control interface module 10c of Fig. 3 does not allow the gas to flow from the first and second outlet ports 14, 16 simultaneously in any of its first, second or third modes. In the first mode, gas may flow from the first outlet port 14 at a preset pressure of approximately 15 psi, and at a preset flow of approximately 12 LPM. In the second mode, gas may flow from the second outlet port 16 at a preset pressure of between approximately 0 psi and approximately 1 psi and a preset flow of approximately 2.5 LPM. These pressure and flow values are, however, specific to only one embodiment and the values thereof may be more or less, depending on the particular application involved. There may be an optional foot switch 26 included which may be provided for substantially similar or identical reasons as described above with respect to Fig. 1

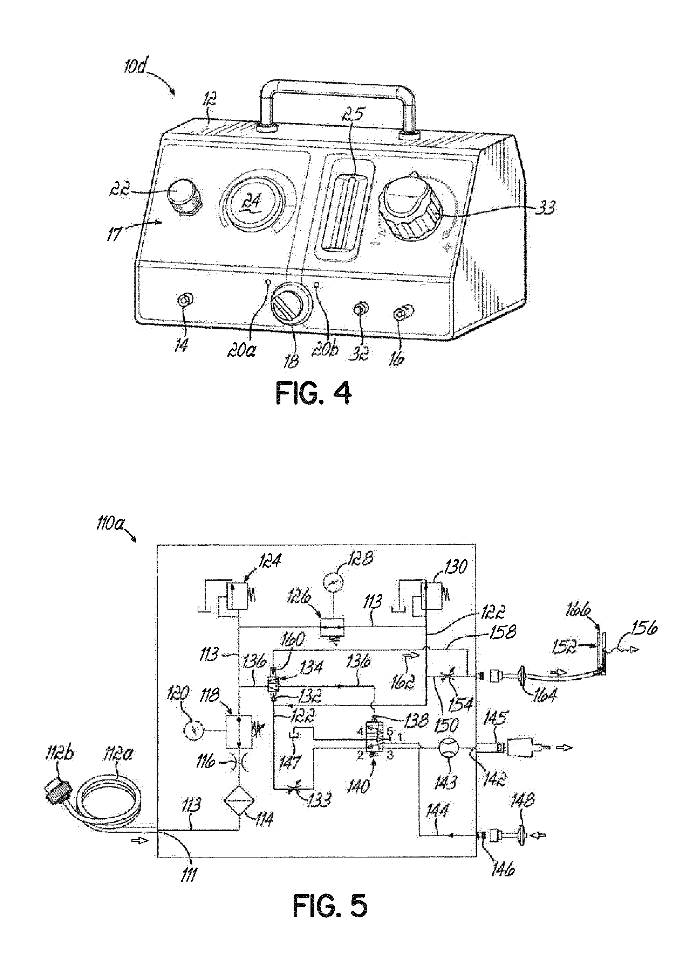

Another alternative embodiment of a control interface module 10d for controlling a regulator (not shown) is shown in Fig. 4. Many of the components the embodiment shown in Fig. 4 of the control interface module 10d are identical or substantially similar to the components described above with reference to the embodiment shown in Figs. 1, 2, and 3, and these components have been marked with the same reference numbers without, in many instances, additional explanation below. Functionally, control interface module 10d is very similar to the embodiment of module 10 shown Fig. 1, module 10b shown in Fig. 2, and module 10c shown in Fig. 3. In that regard, module 10d may be used for controlling operating modes of the regulator. Regulator associated with control interface module 10d only first and second operating modes. However, unlike the embodiment shown in Figs. 1 and 2, the regulator associated with control interface module 10d does not allow the gas to flow from the first and second outlet ports 14, 16 simultaneously. The control interface module 10d is able to be switched only between first and second modes by switch 18 (via positions indicated by points 20a, 20b). In the first mode, pressurized gas may flow from the first outlet port 14 at a flow rate between approximately 0 LPM and approximately 15 LPM, and at pressure of approximately 0 psi to approximately 25 psi. As shown, the flow rate of pressurized gas flowing from the first outlet port 14 in the first mode may not be adjusted using the control interface without adjusting the pressure; however, there may be calibration ports (not shown) in the rear of the control interface module 10d which may allow for adjustment of the flow rate at specific pressures as indicated on pressure gauge 34. A first knob 22 is provided on the module 10 which allows for adjustment of the pressure of the pressurized gas exiting the first outlet port 14 during the first mode. The pressure gauge 34 is also provided so that a user may visualize the pressure of the gas exiting from the first outlet port 14.

In the second mode, gas may flow from the second outlet port 16. The second outlet 16 may be used in applications where the materials are delivered endoscopically or laparoscopically into the body cavity.

There is a second knob 33 provided on control interface module 10d which allows a user to adjust the flow of pressurized gas out of the second outlet 16.

In one embodiment, the flow from the second outlet 16 may be adjusted to be between approximately 0 LPM and approximately 10 LPM. There is a flow meter 35 which provides a visual indication of the flow rate of gas flowing from the second outlet 16.

In each of the embodiments shown in Figs. 1-4, gas travels from a gas source (not shown), to which a connector 38 of a hose assembly 40 is connected. Gas travels through the hose assembly 40 to the input (not shown), where a regulator internal to the control interface modules 10a-d may control the pressure and flow of the gas such that gas flows out of the first and/or second outlet ports 14, 16 at the desired pressure and flow rates, as described above with respect to each embodiment. The regulator(s) embodied, contained, or housed in any of the modules 10a-d may be, but need not be, one of the regulators disclosed below.

Figs. 5A, 6, 7, and 8 show several embodiments of regulators 110a, 110b, 110c, and 110d, respectively. Regulators 110a-d are each represented by a pneumatic circuit that may be embodied in a control interface module, such as, for example, one similar to those described with respect to Figs. 1-4. Because each of the embodiments shown in Figs. 5A, 6, 7, and 8 includes several common structures or features, these common structures or features are marked with the same reference numbers in the figures and described simultaneously herein. In that regard, each of the regulators 110a-d includes a gas inlet 111 connected a hose assembly 112a. The connector 112b of hose assembly 112a is connected to a source of gas (not shown) containing a gas such as Carbon Dioxide (CO₂). Once gas is applied to the inlet 111 a pressurized gas travels into a first line 113 and through an in-line filter 114 which prevents contamination of system components from repeated connection to gas sources and tanks. In one embodiment, the filter 114 is a 0.2 micron filter. Downstream of the filter 114 is a flow restrictor 116, which reduces the flow rate of the gas traveling through the line 122. After traveling through the flow restrictor 116, the gas is applied to a first pressure regulator, which as shown in Figs. 5A, 6, 7, and 8 is a first pressure regulator 118. The first pressure regulator 118 may be set at the factory to drop the pressure from the level applied at the gas inlet 111 (i.e., approximately 200 psi), to a predetermined first pressure, which is preferably between approximately 10 psi and approximately 20 psi. In other words, the first pressure regulator 118 may be a self-relieving pressure regulator which prevents the gas flowing downstream of the first pressure regulator 118 from exceeding the predetermined first pressure. A gauge 120 is coupled to the first pressure regulator 118 in order to provide a visual indication (i.e., on a control interface module) to a user that the pressure output from the first pressure regulator 118 is within the prescribed limits.

Downstream of the first pressure regulator 118 along the first line 113, there is a first safety valve 124 which, in one embodiment, is a pressure relief sequence valve. More specifically, the first safety valve 124 is in fluid communication with the first line 113, downstream of the first pressure regulator 118. The first safety valve 124 is configured to lower the pressure in the first line 113 to a primary threshold pressure, which is approximately within approximately 2-7 psi of the first pressure. In one embodiment, the primary threshold pressure is between approximately 17 psi and approximately 22 psi. The first safety valve 124 is provided in the event that the first pressure regulator 118 should fail. The first safety valve 124, in one embodiment, is a poppet valve that may provide an auditory indication to a user that the pressure flowing in the first line 113 at or near the first safety valve 124 is above the primary threshold pressure.

Further downstream in fluid communication with the first line 113 is a second pressure regulator, which in the embodiments shown in Figs. 5A, 6, 7, and 8 is a second pressure regulator 126. The second pressure regulator 126 may be set at the factory to drop the pressure in the first line 113 to a predetermined second pressure. The second pressure regulator 126 may be a self-relieving pressure regulator. The second pressure regulator 126 includes a pressure gauge 128 coupled thereto in order to provide a visual indication to a user (i.e., on a control interface module) that the pressure output from the second pressure regulator 126 is within prescribed limits. Alternatively, rather than being preset at the factory, the first and second pressure regulators 118, 126 may be calibrated on-site before use of the regulator 110. The second pressure regulator 126 may be a self-relieving pressure regulator, which prevents the gas flowing downstream of the second pressure regulator 126 from exceeding the predetermined second pressure.

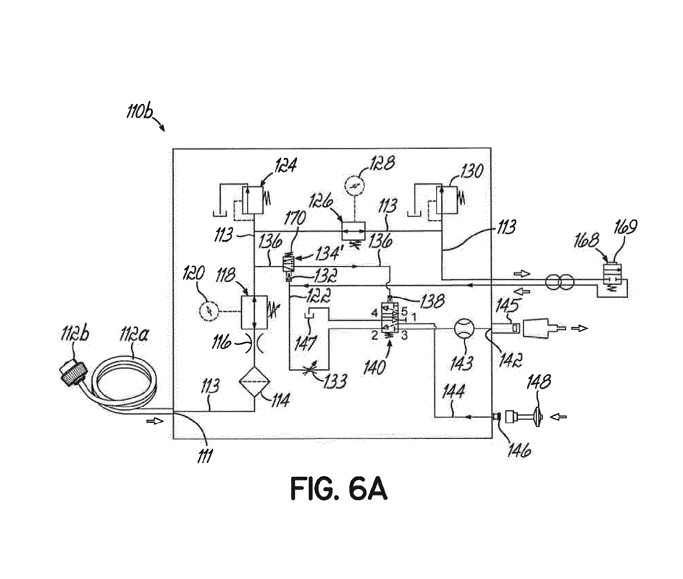

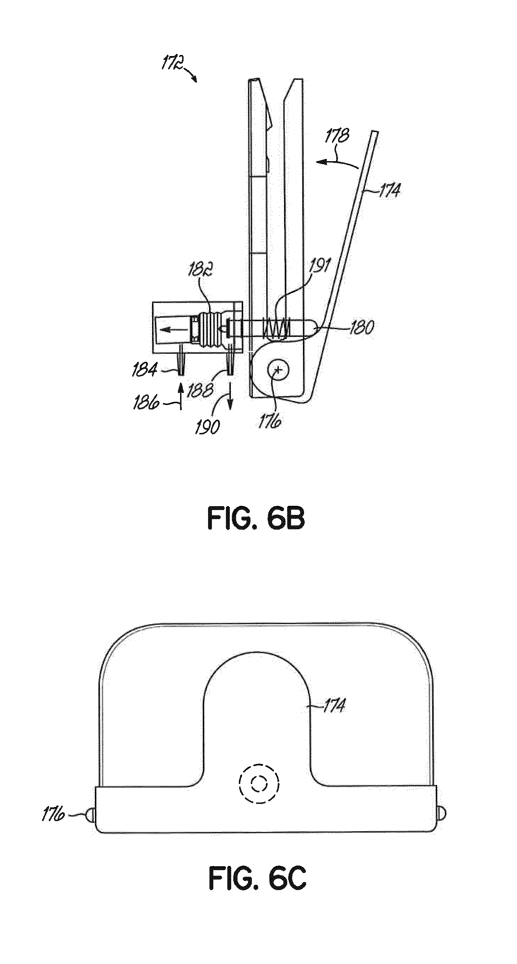

A second safety valve 130 may be included. The second safety valve 130 is downstream of the second pressure regulator 126 and in fluid communication with the first line 113. The second safety valve 130 is configured to lower the pressure in the first line 113 to a secondary threshold pressure. The second safety valve 130, in one embodiment, is a poppet valve that may provide an auditory indication to a user that the pressure flowing in the first line 113 at or near the second safety valve 130 is above the secondary threshold pressure. The secondary threshold pressure may be between the first and second pressures, or between approximately 10 psi and approximately 15 psi. Further downstream, a first pilot valve 132 is in fluid communication with the first line 113. In each embodiment, the first pilot valve 132 may receive a pressure signal in order to actuate the control valve 134 (Fig. 5A) or control valve 134' (Figs. 6-8) from a closed position to an open position. Downstream of the first pilot valve 132 in the first line 113 is a flow control valve 133. When the control valve 134 (or 134') is in the open position, gas at or below the first pressure entering the second line 136 is permitted to travel further downstream in the second line 136 to the second pilot valve 138. Upon receiving a pressure signal at or below the first pressure, the second pilot valve 138 actuates the pneumatic valve 140 from a closed position to an open position. In the open position, the pneumatic valve 140, which, in the embodiments shown, is a 2-position, 5-way valve, permits the gas flowing at or below the second pressure in the first line 113 to travel further downstream to the gas outlet 142. Before exiting from the gas outlet 142, the gas travels through a flow meter 143 which, in one embodiment, is configured to adjust the flow of gas between approximately 0 LPM and approximately 5 LPM. As a safety mechanism, the gas outlet 142 may include a special luer fitting 145 which accepts a male luer such that devices not meant for communication with the outlet 142 are unable to be connected thereto.

Each of the regulators 110a-d (or regulators 210, 310a-b discussed below) disclosed herein may be adapted for use with an applicator for administering liquids to target tissue during a procedure, such as those taught in U.S. Patent No. 7,682,336, issued March 23, 2010, and entitled GAS ASSISTED ENDOSCOPIC APPLICATOR SYSTEM. Alternatively, where applicable, the regulator 110 (or regulator 210 discussed below) disclosed herein may be adapted for use with a fluid-dispensing device, such as those taught in U.S. Patent No. 8,603,025, issued December 10, 2013, and entitled GAS-ASSISTED FLUID-DISPENSING DEVICE.

Vent line 144, in one embodiment, is configured to vent gas from a body cavity at the same rate as gas flowing into the body cavity from a device or applicator connected with the gas outlet 142. Vent line 144 is in fluid communication with the vent line input 146, which may include a filter 148 to prevent materials from entering the regulator 110a-d, thereby preventing the regulator 110 from being tainted. The vent line 144 is configured to operate only when gas is flowing in the first line 113 to the gas outlet 142. In that regard, when the second pilot valve 138 actuates the pneumatic valve 140 from a closed position to an open position, vent line 144 may then communicate with a vent 147. In operation, the vent 147 may vent the gas from the patient in the ambient environment, such as in the operating theatre. However, as discussed below, the vent line 144 may be in fluid communication with a space outside of the adjacent ambient environment (not shown).

The embodiments shown in Figs. 5, 6A, 7, and 8 are particularly advantageous because the pneumatic circuits described therein provide several devices to reduce the pressure and flow rate of gas which is delivered to a device such as an applicator or gas assisted delivery device, and ultimately into a patient. Moreover, while ultimately delivering gas at or below the second pressure to the applicator, the embodiments of the regulator 110a-d as shown in Figs. 5, 6A, 7, and 8 enable gas at the relatively higher first pressure to be ultimately transmitted to the second pilot valve 138 in order to actuate the pneumatic valve 140 into the open position. This configuration particularly advantageous because pneumatic valves, such as the 2-way, 5-position valve 140 shown in Figs. 5, 6A, 7, and 8, may require higher pressure signals in order to be actuated between closed and open positions. Therefore, the configuration allows for transmission of pressurized gas at relatively higher pressure levels to the pneumatic valve 140, while also allowing for dispensing of pressurized gas out of a device (and potentially into a patient) at relatively lower, and safer, pressure levels. Moreover, the embodiments as shown in Figs. 5, 6A, 7, and 8 provide several safety mechanisms to reduce the pressure traveling in the first line 113, and ultimately to an applicator, to a level at or below the second pressure.

Many of the components of each of the embodiments are substantially similar as described above. Each of the individual embodiments may include different features, as described below in a manner such that structures not common across the embodiments are assigned different reference numerals.

With reference to the embodiment shown in Fig. 5A, downstream of the second safety valve 130, there is a bleed line 150 in fluid communication with a bleed vent 152. The bleed line 150 includes a flow control valve 154. The bleed vent 152 is configured to continuously bleed air, as indicated by the arrow 156, when the vent is in the open position. In one embodiment, air bleeds from the bleed vent 152 at a flow rate of approximately -0.2 LPM. Downstream of the point where the first line 113 meets the bleed line 150, the first line 113 is in fluid communication with the first pilot valve 132. There is a return line 158 extending from the bleed line 150 downstream of the flow control valve 154 and in fluid communication with a secondary pilot valve 160. When the bleed vent 152 is prevented from bleeding gas, as will be described below, back pressure builds up through return line 158, thus sending a pressure signal to secondary pilot valve 160. The first and secondary pilot valves 132, 160 are configured to actuate the control valve 134 from the closed to the open position when the first and secondary pilot valves 132, 160 experience differential pressures, as part of a differential pressure switch, as will be appreciated by those skilled in the art.

With continued reference to Fig. 5, the first pilot valve 132 receives gas at or below the second pressure from the first line 113. The secondary pilot valve 160 receives gas at or below the second pressure when the bleed vent 152 is in the open position. When the bleed vent 152 is in the closed position, air is prevented from exiting from the bleed vent 152, resulting in back pressure traveling in the direction opposing arrow 162 in the return line 158. As those skilled in the art will appreciate, due to the difference in pressure received by the first and secondary pilot valves 132, 160, the control valve 134, which is a differential pressure switch, is moved to the open position. When the control valve 134 is in the open position, gas entering the second line 136 is permitted to travel further downstream in the second line 136 to the second pilot valve 138, as disclosed above.

The bleed vent 152 may further include a filter 164. The bleed vent 152 is fluidly connected to a plunger link assembly 166, which is accepted onto a plunger of a syringe (not shown). Air travels through plunger link assembly 166 shown, through the syringe and out of an outlet of the syringe. In this configuration, the bleed vent 152 may be moved to the closed position to thereby create back pressure in the return line 158 in a variety of ways. For example, a practitioner may place his or her finger over the outlet of the syringe to essentially plug, or block, the air from exiting the outlet of the syringe. There are alternative ways of plugging the syringe outlet which will be apparent to those skilled in the art.

Figs. 6A, 7, and 8 show alternative embodiments of a regulator 110b, 110c, and 110d, respectively. In each of these embodiments, downstream of the second safety valve 130 is a mechanically actuatable switch valve 168 in fluid communication with the first line 113. In a closed position, the mechanically actuatable switch valve 168 prevents the gas from flowing downstream in the first line 113. By moving the switch valve 168 to an open position (by depressing button 169), gas is permitted to flow downstream of the switch valve 168 at or below the second pressure.

Thereafter, the first pilot valve 132 receives a pressure signal at a level at or below the first pressure, thereby actuating the control valve 134' into the open position. In one embodiment, the control valve 134' is actuatable into the open position by a pressure signal of at least approximately 5 psi to the first pilot valve 132. As shown in Figs. 6A, 7, and 8, the control valve 134' may be movable to the closed position by a spring return 170. The spring return 170 is configured to return the first pilot valve 132 to the closed position when the first pilot valve 132 is no longer receiving pressurized gas, or is receiving pressurized gas providing a force that is insufficient to overcome the force from spring return 170. As disclosed above, after the control valve 134' has moved to the open position, gas at or below the first pressure entering the second line 136 is permitted to travel further downstream in the second line 136 to the second pilot valve 138, which actuates the pneumatic valve 140 into the open position.

In the embodiments shown in Figs. 6A, 7, and 8, the mechanically actuatable switch valve 168 may be configured to be a foot switch. When the button 169 is depressed, the switch valve 168 moves to the open position, thereby allowing the first pilot valve 132 to receive a pressure signal at a level at or below the first pressure, thereby actuating the control valve 134 into the open position. Persons skilled in the art will appreciate that the mechanically actuatable switch valve 168 may include any mechanically actuatable valve which moves to an open position to allow the passage of gas when a portion, such as a button 169, is actuated or depressed.

Figs. 6B and 6C show an alternative embodiment of a mechanically actuatable switch valve, shown as a plunger link switch assembly 172. In Fig. 6B, plunger link switch assembly 172 in a second position which prevents gas prevents gas from traveling downstream in first line 113. The plunger link switch assembly 166 includes a lever 174 rotatable about pivot 176. The actuation of lever 174 in a direction of arrow 178 pushes the plunger rod 180 towards the check valve 182, which in one embodiment may be a poppet valve. The axial movement of the plunger rod 180 causes a ball seat (not shown) to be moved from a first position where the ball seat is blocking air from flowing through the first barbed fitting 184 in the direction of arrow 186 and through the valve 182, to a second position. In the second position, the ball seat permits air to flow through the valve 182 and out from the second barbed fitting 188 in the direction of arrow 190. The first barbed fitting 184 is in fluid communication with an upstream portion of the first line 113. The second barbed fitting 188 is in fluid communication with a downstream portion of the first line 113. Air flowing out of the second barbed fitting 188 flows downstream thereof in the first line 113 at or below the second pressure to the first pilot valve 132, thereby actuating the control valve 134 into an open position, as described above. The check valve 182, in the embodiment shown, may be a poppet valve, the operation of which will be apparent to those skilled in the art. Upon release of the lever 174, spring 191 returns the plunger rod 180 and thus the lever 174 to the position shown in Fig. 6B.

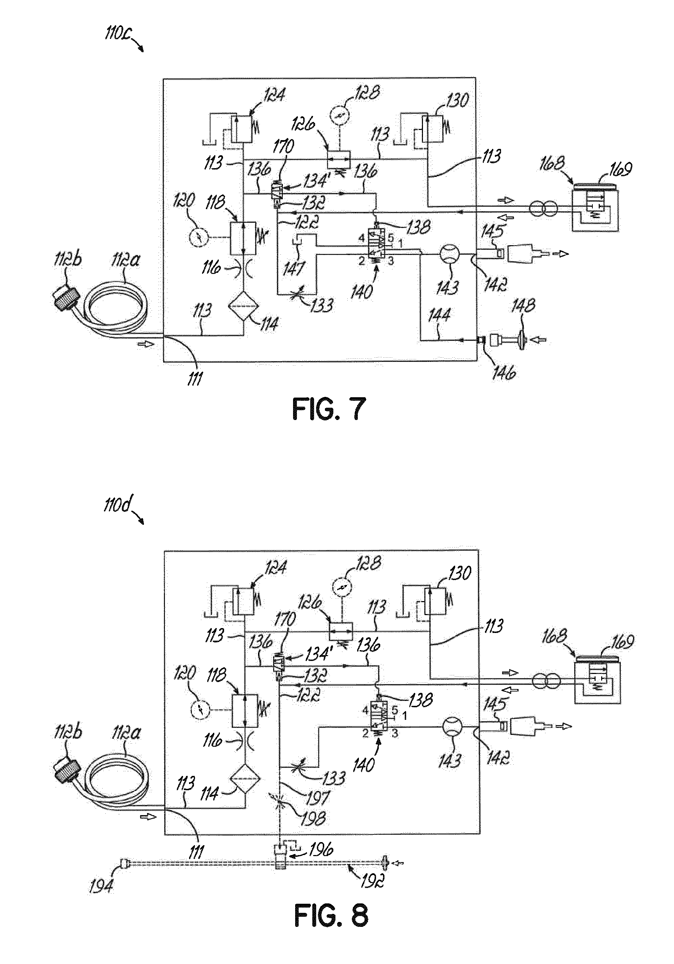

The embodiment shown in Fig. 8 includes an alternative embodiment of a vent line 192. The vent line 192 may be in fluid communication with a vent outlet 194 that leads to a space external to the ambient environment, such as the operating theatre. Venting outside of the operating theatre may be desirable where materials being delivered by the applicator may be caustic or generally harmful, such as cancer drugs which may include radioactive materials. Moreover, electrocautery may be utilized during a procedure, which may cause the buildup of smoke in the abdominal cavity, particularly during laparoscopic procedures. Venting outside of the operating theatre may prevent such materials and smoke, for example, from entering the operating theatre. Furthermore, the embodiment as shown in Fig. 7 prevents the harmful materials from traveling through and possibly tainting the regulator 110.

Still referring to Fig. 8, gas entering the patient vent line 192 is able to travel to the vent outlet 194 when a pinch valve 196, which is in fluid communication with the first line 113, receives gas from the first line 113. In between the first line 113 and the pinch valve 196 is a third line 197, which includes a flow control valve 198 in fluid communication with the third line 197. The flow control valve 198 may delay the spring-loaded opening and closure of the pinch valve 196. In addition to being fluidicly communicated with an external environment, the vent line 192 may include a vacuum extraction system (not shown). The alternative embodiment of a vent line 192 as shown in Fig. 8 is not limited to the embodiment of only Fig. 8. Rather, it is anticipated that the embodiments shown in Figs. 5, 6A, and 7, as well as other alternative embodiments, may be configured to utilize vent line 192 shown in Fig. 8.

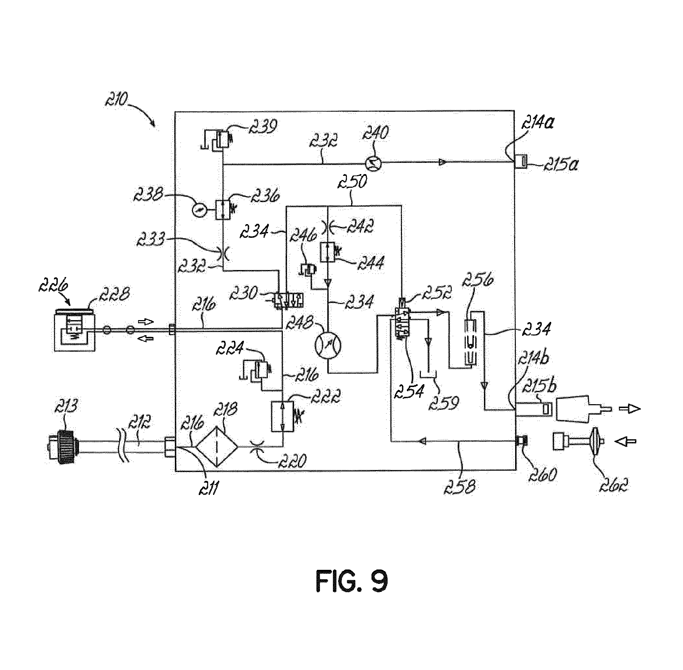

Fig. 9 shows another embodiment of a regulator 210. The regulator 210 is configured to regulate the pressure and flow of gas a pressurized gas flowing into gas inlet 211 connected a hose assembly 212. The connector 213 of hose assembly 212 is connected to a source of gas (not shown) containing a gas such as Carbon Dioxide (CO₂). More particularly, the regulator 210 regulates the pressure and flow of gas flowing between inlet between the gas inlet 211 and first and second gas outlets 214a, 214b. As shown, the regulator 210 is represented by a pneumatic circuit that may be embodied, contained, or housed in a control interface module, such as, control interface modules 10a-d (Figs. 1-4) described hereinabove. The outlets 214a, 214b may have special luer fittings 215a, 215b (acting as first and second gas outlet ports, for example) connected thereto for connection to tubing or gas-assisted applicators as described herein.

Once gas is applied to the inlet 211, pressurized gas travels through a first line 216 to an in-line filter 218, which prevents contamination of system components from repeated connection to gas sources and tanks. In at least one embodiment, the filter 218 is a 200-mesh stainless steel filter. Downstream of the filter 218 is a flow restrictor 220, which reduces the flow rate of the gas traveling through the flow restrictor 220. Downstream of the flow restrictor 220 on the first line 216, the gas flows past to a first pressure regulator, which is a first pressure regulator 222. The first pressure regulator 222 may be set at the factory to drop the pressure from the level applied at the inlet 211 (i.e., approximately 100 psi), to a first pressure, which is preferably between approximately 25 psi and approximately 35 psi. In other words, the first pressure regulator 222 may be a self-relieving pressure regulator which prevents the gas flowing downstream of the first pressure regulator 222 from exceeding the main pressure.

There is a first safety valve 224 which, in one embodiment, is a pressure relief sequence valve. More specifically, the first safety valve 224 is in fluid communication with the first line 216, downstream of the first pressure regulator 222. The first safety valve 224 is configured to lower the pressure in the first line 216 to a threshold pressure in the event that the first pressure regulator 222 fails. The threshold pressure may be approximately within approximately ± 2-7 psi of the first pressure. In one embodiment, the primary threshold pressure is between approximately 30 psi and approximately 40 psi. The first safety valve 224, in one embodiment, is a poppet valve that may provide an auditory indication to a user that the pressure flowing in the first line 216 at or near the first safety valve 224 is above the threshold pressure.

Further downstream is a switch valve 226 having open and closed positions. In the closed position, pressurized gas is prevented from flowing downstream from the switch valve 226. When the switch valve 226 is in the open position, the pressurized gas is permitted to flow downstream from the switch valve 226. The switch valve 226 may be a foot or other type of mechanically actuated switch such that when a user depresses the button 228, the switch valve 226 is in the open position and pressurized gas is permitted to flow downstream therefrom or thereof. Downstream of the switch valve 226 is a multiple position valve 230. In the embodiment shown, the multiple position valve 230 is a two-position, four-way, five port valve that is moveable between first and second positions. The valve 230 may be movable between first and second positions by a switch, handle or knob such as a switch that may be on a control interface module (Figs. 1-4) as described hereinabove. When the valve 230 is in the first position, pressurized gas may flow into a second line 232. When the valve 230 is in the second position, pressurized gas is permitted to flow into a third line 234. In a third position, the valve 230 allows the gas to flow into both of the second and third lines 232, 234. The second line 232 is essentially a first supply line that leads to the first outlet 214a, while the third line 234 is essentially a second supply line that leads to the second outlet 214b.

Gas flowing into the second line 232 flows past a flow restrictor 233, which may reduce the flow rate of the gas flowing in the second line 232. Then, the gas flows past a second pressure regulator, such as the second pressure regulator 236 as shown. The second pressure regulator 236 is provided so that gas flowing downstream therefrom does not exceed a second pressure. The second pressure may also be referred to herein as the first supply pressure since the second line 232 (or first supply line) 232 leads to the first outlet 214a, such that gas flowing out of first outlet 214a is at the second pressure. There is a pressure gauge 238 coupled with the second pressure regulator 236 which provides a visual indication of the pressure in the first line 232 (i.e., on a control interface module), at the second pressure regulator 236. In one embodiment, the second pressure regulator 236 may be preset at a specific pressure. However, in other embodiments, the second pressure regulator 236 may be adjustable by a handle or knob of a control interface module, such as, for example, one similar to the embodiments as described hereinabove. The second pressure may be adjustable between approximately 0 psi and approximately 30 psi such that the pressure of the gas exiting from the first outlet 214a is between approximately 0 psi and approximately 30 psi. The second pressure may be according to the preferences of the user and of the specific requirements of the procedure or action being performed.

Downstream in the first supply line 232 there is a second safety valve 239. The second safety valve 239 is a pressure relief valve and is provided in the event that one or both of the pressure regulators 222, 236 fails. In one embodiment, the pressure relief valve 239 is configured to lower the pressure in the first supply line 232 to within approximately ±2-7 psi of the first supply pressure (i.e., if one of the pressure regulators 222, 236 fails). Further downstream of the pressure relief valve 239 is a flow control valve 240. The flow control valve 240 may be adjustable between approximately 0 LPM and approximately 15 LPM such that the flow of the pressurized gas flowing from the first outlet 214a may be in that range at a given pressure. The flow control valve 240 may or may not be adjustable by a knob, for example, on a control interface module as described herein.

When the multiple position valve 230 is in the second position, pressurized gas is permitted to enter the third line (or second supply line) 234. Gas flowing into the third line 234 is applied to a flow restrictor 242, which reduces the flow rate of the gas traveling downstream the flow restrictor 242. Downstream of the flow restrictor 242, the gas is applied to a third pressure regulator, such as the third pressure regulator 244 as shown. The third pressure regulator 244 is provided so that gas flowing downstream therefrom or thereof does not exceed a second supply pressure. In one embodiment, the third pressure regulator 244 may be preset at a specific pressure. However, in other embodiments, the third pressure regulator 244 may be adjustable by a handle or knob of a control interface module, such as, for example, one described hereinabove. The third pressure may be adjustable between approximately psi 0 and approximately 15 psi such that the pressure of the gas exiting from the second outlet 214b is between approximately 0 psi and approximately 15 psi. The third pressure may be adjusted according to the preferences of the user and of the specific requirements of the procedure or action being performed. Downstream of the third pressure regulator 244 is a third safety valve 246. The third safety valve 246 is provided in the event that one or both of the pressure regulators 222, 244 fails. In one embodiment, the third safety valve 246 is configured to lower the pressure in the third line 234 to within approximately ±2-7 psi of the second supply pressure.

Further downstream, there is a flow control valve 248, which may be adjustable by a knob or other mechanism 33 on, for example, a control interface module. In one embodiment, the flow control valve 248 is adjustable such that the pressurized gas may flow downstream therefrom or thereof at flow rate between approximately 0 LPM and approximately 10 LPM.

There is an auxiliary line 250 extending from the third line 232 upstream of the third pressure regulator 244. On the auxiliary line 250, downstream from where the auxiliary line 250 extends from the second supply line 232, there is pilot valve 252. The pilot valve 252 is in fluid communication with the auxiliary line 250. Due to its position upstream of the third pressure regulator 244, the auxiliary line 250 receives pressurized gas at the first pressure, rather than at the third pressure. Thus, the first pressure is applied to pilot valve 252 when the multiple position valve 230 is in the second position. When the main pressure is applied to the pilot valve 252, the control valve 254 actuates into an open position. The control valve 254 provided herein is beneficial for several reasons. Because it is actuatable at the first pressure, which may be between approximately 25 and 30 psi, less precise pressures are needed in order to actuate the control valve 254. For example, pneumatic control valves which actuate between and among positions at lower pressures require more precise and controlled delivery of pressurized gas. Due to the precision needed in, and the sensitivity of, valves requiring lower pressure, the cost may be higher in those situations than that of the control valve 254 disclosed herein. Moreover, the control valve 254 may be a stock item, rather than having to be specially or custom manufactured. Furthermore, the configuration allows for transmission of pressurized gas at relatively higher pressure levels to the control valve 254, while also allowing for dispensing of pressurized gas out of a device (and potentially into a patient) at relatively lower, and safer, pressure levels.

When the control valve 254 is in the open position, gas flowing through the third line 234 is able to continue past the control valve 254 and to the second gas outlet 214b. In between the control valve 254 and the outlet 214b, however, there may be flow sensor (not shown) which may communicate with a flow meter 256, which may be shown, for example, on a control panel of a control interface module as described herein.

The second outlet 214b may be used in applications where at least one material is delivered endoscopically or laparoscopically into the body cavity under the aid of the gas. In those applications, a vent line 258 and a vent 259 may be used for purposes of counteracting the increased volume and/or pressure of gas inside the body cavity. More specifically, vent line 258 operates when gas is flowing through the second outlet 214b. The vent line 258 may also be controlled by the control valve 254 such that when the control valve 254 is in the open position, gas to be vented may travel into the vent line 258 and out of the vent 259. The vent line 258 also includes a vent line input 260 and a vent filter 262, in order to prevent contamination of the regulator 210.

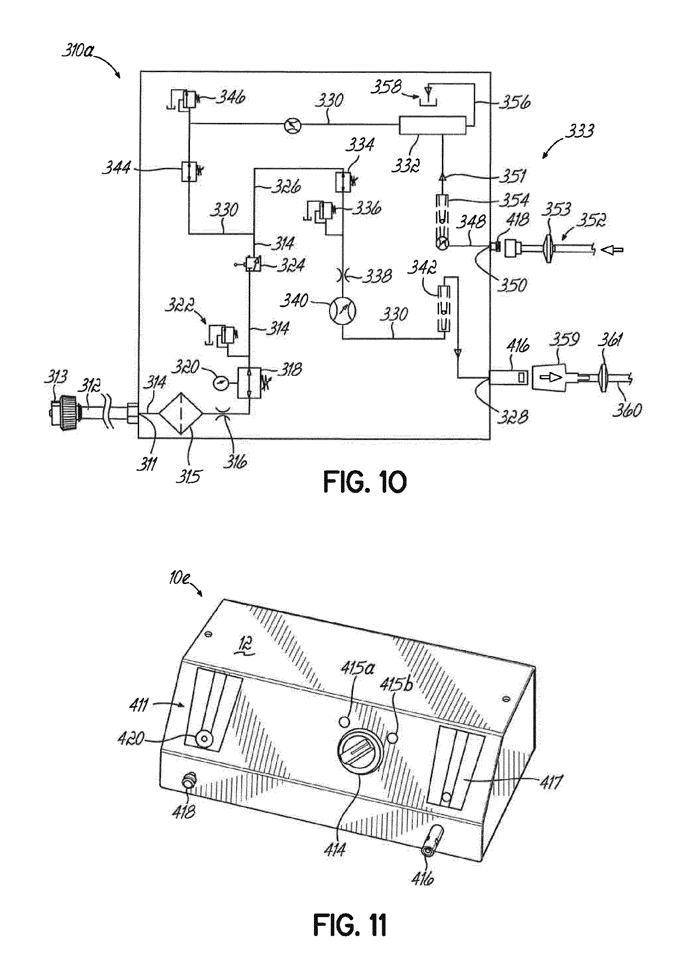

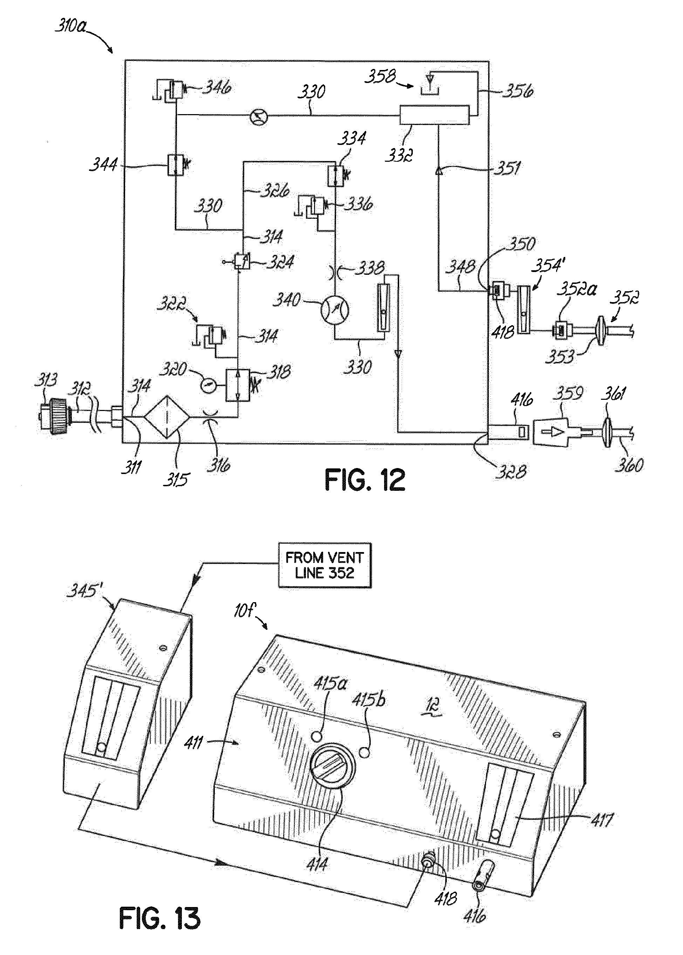

Figs. 10 and 12 show different embodiments of regulators 310a and 310b, respectively. Regulators 310a, 310b are each represented by a pneumatic circuit that may be contained or housed within a control interface module. Specifically, in one embodiment, the regulators 310a, 310b, may be contained or housed within control interface modules 410 described below with respect to Figs. 11 and 13, respectively. Because each of the embodiments includes several common structures or features, these common structures or features are marked with the same reference numbers in the figures and described simultaneously hereinbelow. In that regard, each of the regulators 310a, 310b includes a gas inlet 311 connected a hose assembly 312. The connector 313 of hose assembly 312 is connected to a source of gas (not shown) containing a pressurized gas such as Carbon Dioxide (CO₂). Once gas is applied to the inlet 311 a pressurized gas travels through a main line 314 to an in-line filter 315 which prevents contamination of system components from repeated connection to gas sources and tanks. In one embodiment, the filter 315 is a 0.2 micron filter. Downstream of the filter 315 is a flow restrictor 316, which reduces the flow rate of the gas traveling through the flow restrictor 316. After traveling through the flow restrictor 316, the gas is applied to a first pressure regulator, which as shown in Figs. 10 and 12, and is a first pressure regulator 318. The first pressure regulator 318 may be set at the factory to drop the pressure from the level applied at the gas inlet 111 (i.e., approximately 200 psi), to a predetermined first pressure, which is preferably between approximately 10 psi and approximately 40 psi. A gauge 320 is coupled to the first pressure regulator 318 in order to provide a visual indication (i.e., on a control interface module) to a user that the pressure output from the first pressure regulator 318 is within the prescribed limits.

There is a first safety valve 322 downstream of the first pressure regulator 318. In one embodiment, the first safety valve 322 is a pressure relief sequence valve. The first safety valve 322 is configured to lower the pressure in the main line 314 to a primary threshold pressure, which is within approximately ±2-7 psi of the first pressure if, of course, the first pressure regulator 318 should fail. In one embodiment, the primary threshold pressure is between approximately 17 psi and approximately 22 psi. The first safety valve 322, in one embodiment, is a poppet valve that may provide an auditory indication to a user that the pressure flowing in the first line 314 at or near the first safety valve 322 is above the primary threshold pressure. Downstream of the first safety valve 322 is a one position, three-way control valve 324 that is movable between open and closed positions upon the actuation of a switch on, for example, a control interface module 410a, such as switch 414 described in the embodiment shown in Fig. 11. In the closed position, control valve 324 prevents pressurized gas from flowing further downstream. However, in the open position, control valve 324 allows pressurized gas to flow further downstream in the main line 314. Just downstream of the control valve 324, main line 324 essentially bifurcates into two lines, supply line 326 (also referred to herein as "first line"), which ultimately leads to outlet 328, and a vacuum assist line 330 (also referred to herein as "second line"), which ultimately leads to vacuum generator 332 (or vacuum extraction system) that provides a vacuum for a vent system 333 (described below). Thus, gas may flow simultaneously into the supply line 326 and vacuum assist line 330.

Regarding supply line 326, there is a second pressure regulator in fluid communication with the supply line 326, which, in the embodiments shown in Figs. 11 and 13, is a second pressure regulator 334. The second pressure regulator 334 may be set at the factory to drop the pressure in the vacuum supply line to a predetermined second pressure level, which may be between approximately 0 psi and approximately 20 psi, and is preferably between approximately 10 psi and approximately 15 psi. The second pressure regulator 334 may be a self-relieving pressure regulator. The second pressure regulator 334 may be a self-relieving pressure regulator, which prevents the gas flowing downstream of the second pressure regulator 334 from exceeding the predetermined second pressure level.

A second safety valve 336 may also be included. The second safety valve 336 is downstream of the second pressure regulator 334 and in fluid communication with the supply line 326. The second safety valve 336 is configured to lower the pressure in the supply line 326 to a secondary threshold pressure, which is within approximately ± 2-7 psi of the second pressure, in the event that the first pressure regulator 318 and/or the second pressure regulator 334 should fail. The second safety valve 336, in one embodiment, is a poppet valve that may provide an auditory indication to a user that the pressure flowing in the supply line 326 at or near the second safety valve 336 is above the secondary threshold pressure. Further downstream along the supply line 326 (i.e., in fluid communication with the supply line) is a flow restrictor 338 and a flow control valve 340. Flow restrictor 338 is configured to reduce the flow rate of gas flowing in the supply line 326. Flow control valve 340, similarly, is configured to reduce the flow rate in the supply line 326 to a rate of between approximately 0 LPM and approximately 10 LPM. Downstream of the flow control valve 340 is a flow meter 342 which determines the flow rate of the gas flowing from outlet 328. In that regard, flow meter 342 is configured to adjust the flow rate of gas flowing in the supply line 326 to between approximately 0 LPM and approximately 5 LPM (preferably approximately 4.5 LPM) so that the gas supplied to a downstream device or applicator is within such a range of flow rate and at the second pressure level.

Still referring to Figs. 10 and 12, and regarding the vacuum assist line 330, there is a third pressure reducing device in fluid communication with the vacuum assist line 330, which, in the embodiments shown in Figs. 11 and 13, is a third pressure regulator 344. The third pressure regulator 344 may be set at the factory to drop the pressure in the vacuum assist line 330 to a predetermined third pressure, which may be between approximately 0 psi and approximately 20 psi. The second pressure regulator 344 may be a self-relieving pressure regulator. The first, second, and third pressure regulators 318, 334, 344 may be preset at the factory, or may be calibrated on-site before use of the regulators 310a, 310b. The third pressure regulator 344 may be a self-relieving pressure regulator, which prevents the gas flowing downstream of the third pressure regulator 344 from exceeding the predetermined third pressure level.

A third safety valve 346 may also be included. The third safety valve 346 is downstream of the third pressure regulator 344 and in fluid communication with the vacuum assist line 330. The third safety valve 346 is configured to lower the pressure in the vacuum assist line 330 to a tertiary threshold pressure level, which is within approximately ± 2-7 psi of the second pressure, in the event that the first pressure regulator 318 and/or the third pressure regulator 344 should fail. The third safety valve 346, in one embodiment, is a poppet valve that may provide an auditory indication to a user that the pressure flowing in the vacuum assist line 330 at or near the third safety valve 346 is above the third pressure level. The third pressure level may be between the first and second pressures, or between approximately 10 psi and approximately 15 psi.

Downstream of the second safety valve 336 is a flow control valve 340. Flow control valve 340 is operable to adjust the flow rate of gas flowing in the vacuum supply line to a rate of between approximately 5 LPM and approximately 10 LPM. Further downstream of the flow control valve 340 is the vacuum generator 332 which is part of a vent system 333. Vent system 333 may be used for purposes of counteracting the increased volume or pressure of gas inside the body cavity when pressurized gas is directed into the body cavity during, for example, a laparoscopic or endoscopic procedure using a gas-assisted delivery device. When the vacuum generator 332 receives pressurized air from the vacuum assist line 330, it may to create negative pressure within portions of vent system 347. More particularly, the vacuum generator 332 creates a vortical air flow that creates a negative pressure within a first internal vent line 348. The negative pressure within first internal vent line 348 draws air into the first internal vent line 348 through vent port 350, from external vent line 352 (which, in use, would be in fluid communication with a body cavity). Air traveling into the first internal vent line 348 travels in the direction of arrow 351, through the vacuum generator 332 and into second internal vent line 356, and eventually out of vent 358. Vent 358 may lead to the ambient environment, such as in the operating theatre. However, as discussed below, the vent 358 may be in fluid communication with a space outside of the adjacent ambient environment (not shown).

In the embodiment shown in Fig. 10, first internal vent line 348 of regulator 310a includes a flow meter 354 with a flow control valve 356 in fluid communication therewith and along the first internal vent line 348. Flow meter 354 is included within the fluidic circuit of regulator 310a such that where the regulator 310a is essentially housed within a control interface module 410a (Fig. 11), the flow meter 354 is mounted internally, such that it is within the housing 412 of control interface module 410a (Fig. 11). Flow meter 354 is adjustable such that the gas flowing downstream from the flow meter 354 towards vacuum generator 332 at a flow rate of between approximately 0 LPM and approximately 5 LPM.

The regulator 310a of Fig. 10 is associated with control interface module 410a shown in Fig. 11 such that the various controls on control panel 411, described below, are configured to control operation of certain portions of the regulator 310a. Input port (not shown), that is in fluid communication with inlet 311 of regulator 310a, is not shown in Fig. 11 but may be in any portion of the module 410b, preferably a rear portion.

Module 410a also includes a connection port 418 to which the external vent line 352 (including filter 353) may be connected. For adjusting flow meter 354 (Fig. 11A), module 410 includes an adjustment knob 420. As shown, knob 420 is slidable such that sliding the knob 420 may increase or decrease the flow rate of gas downstream of the flow meter 354 in first internal vent line 348 as described herein.

The regulator 310b of Fig. 12 is associated with control interface module 410b shown in Fig. 13 such that the various controls, described below, are configured to control operation of certain portions of the regulator 310b. Many of the components the embodiment shown in Fig. 13 of the control interface module 410b are identical or substantially similar to the components described above with reference to the embodiment shown in Fig. 11, and these components have been marked with the same reference numbers without additional explanation below. Similarly, input port, which is in fluid communication with inlet 311 of regulator 310a, is not shown in Fig. 11 but may be in any portion of the module 410, preferably a rear portion. Regulator 310b includes an external flow meter 354'. As shown in Figs. 12 and 13, flow meter 354' is positioned between connection port 418 and external vent line 352 (and vent line connection 352a). Therefore, module 410b does not include a control knob for adjusting flow meter 354. Rather, flow meter 354' is preferably preset such that the flow of gas entering the first internal vent line 348 is between approximately 0 LPM and approximately 5 LPM, but preferably approximately 4.5 LPM. In operation, in one embodiment, outlet port 416 would be fluidly communicated with a laparoscopic or endoscopic gas assisted device (via special luer fitting 359 and line 360, including filter 361). Connection port 418 would be fluidly communicated with external vent line 352, which would be in fluid communication with a body cavity.

In order to begin the procedure, a practitioner would direct at least a distal portion of the gas-assisted applicator into a body cavity of a patient. Furthermore, a distal portion (not shown) of external vent line 352 would be fluidly communicated with the body cavity (before, after, or concurrently with the direction of the device into the body cavity). Once the switch 414 is moved to the second position such that gas may flow through the regulators 310a, 310b as described above, thereby allowing gas to flow into the body cavity via the gas-assisted applicator, and also allowing gas to be vented from the body cavity under assistance from the vent system, as described herein.

While the present invention has been illustrated by the description of specific embodiments thereof, and while the embodiments have been described in considerable detail, additional advantages and modifications will readily appear to those skilled in the art. The various features discussed herein may be used alone or in any combination.

Embodiments of the invention can be described with reference to the following numbered clauses, with preferred features laid out in the dependent clauses:
1. A control interface module configured to control an internal regulator for regulating a pressure and flow of a pressurized gas flowing from a gas inlet to first and second gas outlets, comprising:
   a housing having a gas inlet and first and second gas outlets; and
   a multiple position switch on the housing, the switch configured to control whether the gas is to permitted to flow from only one of, each of, or neither of the first and second outlets.
2. The control interface module of clause 1, wherein the switch includes first, second, and third positions, wherein each position of the switch corresponds to a different mode of operation, wherein:
   in a first mode, gas is permitted to flow out of only one of the first or second outlets; and
   in a second mode, gas is permitted to flow out of each of the first and second outlets.
3. The control interface module of clause 2, wherein:
   in a third mode, gas is prevented from flowing from either of the first and second outlets.
4. The control interface module of clause 1, further comprising:
   a knob configured to adjust pressure of the gas flowing from at first outlet.
5. The control interface module of clause 1, further comprising:
   a mechanically actuatable switch including a toggle switch having on and off positions, wherein:
      when the toggle switch is in the on position, gas is permitted to flow continuously out of the first gas outlet; and
      when the toggle switch is in the off position, gas is permitted to flow out of the first outlet upon actuation of the mechanically actuatable switch.
6. A system for gas assisted delivery of at least one material, comprising:
   the control interface module of clause 1; and
   an applicator.
7. A method of performing a gas-assisted procedure using the control interface module of clause 1, comprising:
   fluidly communicating the inlet with a source of pressurized gas;
   fluidly communicating at least one of the first or second outlets with an applicator; and
   operating the switch to permit gas to flow out of at least one of the first or second outlets, corresponding to which of the first or second outlets is fluidly communicated with the applicator.
8. The method of clause 7, further comprising:
   fluidly communicating only one of the first or second outlets with a applicator; and
   dispensing the one or more materials from the applicator onto a treatment site.
9. The method of clause 8, wherein dispensing the at least one material from the applicator further comprises:
   delivering the one or more materials into a body cavity endoscopically or laparoscopically.
10. A regulator for regulating the pressure and flow of gas a pressurized gas traveling between a gas inlet and a gas outlet, comprising:
   a first pressure regulator in fluid communication with the gas inlet and configured to prevent the gas flowing downstream of the first pressure regulator in the first line from exceeding a first pressure
   a first line between the first pressure regulator and the gas outlet;
   a second pressure regulator in fluid communication with the first line, downstream of the first pressure regulator, and configured to prevent the gas flowing in the first line downstream of the second pressure regulator from exceeding a second pressure;
   a first pilot valve in fluid communication with the first line;
   a control valve actuatable to an open position when gas is transmitted to the first pilot valve at or below the second pressure;
   a second pilot valve in fluid communication with the first line such that gas is transmitted at or below the first pressure to the second pilot valve when the control valve is in the open position; and
   a pneumatic valve in fluid communication with the first line and moveable to an open position when gas at or below the first pressure is transmitted to the second pilot valve, the pneumatic valve in the open position permitting the gas in the first line to exit through the gas outlet at or below the second pressure.
11. The regulator of clause 10, further comprising:
   a second line extending from the first line to the second pilot valve, thereby fluidly communicating the first line and the second pilot valve.
12. The regulator of clause 11, wherein the control valve is in fluid communication with the second line at a point between the first line and the second pilot valve.
13. The regulator of clause 11, wherein the second line extends from the first line at a point between the first and second pressure regulators.
14. The regulator of clause 10, further comprising:
   at least a first safety valve in fluid communication with the first line and configured to reduce pressure of the gas flowing in the first line to an amount between the first and second pressures in the event of a failure of one or both of the first and second pressure reducing devices.
15. The regulator of clause 10, wherein:
   the first pressure is between approximately 10 psi and approximately 20 psi; and
   the second pressure is between approximately 7 psi and approximately 10 psi.
16. The regulator of clause 10, wherein the control valve is defined by a differential pressure switch.
17. The regulator of clause 10, further comprising:
   a mechanically actuatable switch valve in fluid communication with the first line, upstream of the first pilot valve, the switch valve including an open position where gas is permitted to flow downstream in the first line and a closed position where gas is prevented from flowing downstream in the first line.
18. The regulator of clause 17, wherein the switch valve is defined by a plunger link switch assembly.
19. The regulator of clause 17, wherein the switch valve includes a foot switch.
20. The regulator of clause 10, further comprising:
   a vent line in fluid communication configured to vent an amount of gas from a body cavity in fluid communication with the regulator.
21. A method of regulating a pressure and flow of a pressurized gas traveling between a gas inlet and a gas outlet of a regulator, comprising:
   introducing pressurized gas into a first line of the regulator from an inlet of the regulator;
   reducing the pressure of the gas to a first pressure using a first pressure regulator, thereby preventing gas flowing downstream of the first pressure regulator in the first line from exceeding the first pressure;
   reducing the pressure of the gas flowing in the first line to a second pressure using a second pressure regulator, thereby preventing gas flowing downstream of the second pressure regulator in the first line from exceeding the second pressure;
   transmitting the pressurized gas at or below the second pressure to a first pilot valve, thereby actuating a control valve to an open position;
   transmitting the gas at or below the first pressure to a second pilot valve, thereby actuating a pneumatic valve to an open position; and
   permitting the pressurized gas to flow downstream of the pneumatic valve in the first line and thereby flow out of the gas outlet when the pneumatic valve is in the open position.
22. The method of clause 21, wherein the regulator further comprises a second line extending from the first line to the second pilot valve, and the method further comprises:
   transmitting the gas from the first line to the second line at or below the first pressure to the second pilot valve, thereby actuating the pneumatic valve to the open position.
23. The method of clause 21, wherein:
   the first pressure is between approximately 10 psi and approximately 20 psi; and
   the second pressure is between approximately 7 psi and approximately 10 psi.
24. The method of clause 21, wherein the regulator further comprises a mechanically actuatable switch valve in fluid communication with the first line, upstream of the first pilot valve, and the method further comprises:
   actuating the switch between an open position where gas is permitted to flow downstream in the first line, and a closed position where gas is prevented from flowing downstream in the first line.
25. A regulator for use with a gas-assisted applicator in an endoscopic or a laparoscopic procedure whereby the device is in fluid communication with a body cavity, comprising:
   an inlet configured to be fluidly communicated with a source of pressurized gas;
   an outlet in fluid communication with the inlet and configured to be coupled with an applicator; and
   a vent system including a vent line leading to a vent outlet, and a vacuum extraction system in fluid communication with the vent line, wherein the vent line is configured to be fluidly communicated with the body cavity such that gas within the body cavity may be drawn into the vent line and out of the vent outlet during operation of the vacuum extraction system.
26. A method of venting a body cavity during a laparoscopic or endoscopic procedure using a regulator comprising an inlet configured to be fluidly communicated with a source of pressurized gas, an outlet configured to be coupled with a gas-assisted endoscopic or laparoscopic applicator, the outlet in fluid communication with the inlet such that gas flowing into the inlet may flow to and be dispensed by the device into the body cavity, and a vent system including a vent line leading to a vent outlet, and a vacuum extraction system in fluid communication with the vent line, the method comprising:
   fluidly communicating the inlet to a source of pressurized gas;
   fluidly communicating the vent line to the body cavity; and
   operating the vacuum extraction system such that gas within the body cavity may be drawn through the vent line and out of the vent outlet.
27. A method of performing a gas-assisted endoscopic or laparoscopic procedure using a gas-assisted applicator and a regulator, the regulator comprising an inlet, an outlet fluidly communicating with the inlet such that gas flowing into the inlet may flow to the outlet, and a vent system including a vent line leading to a vent outlet, and a vacuum extraction system in fluid communication with the vent line, the method comprising:
   fluidly communicating the inlet to a source of pressurized gas;
   fluidly communicating the vent line with the body cavity;
   fluidly communicating the gas-assisted applicator with the outlet;
   directing the gas-assisted applicator into the body cavity;
   directing gas into the body cavity from the gas-assisted applicator; and
   operating the vacuum extraction system such that gas within the body cavity may be drawn through the vent line and out of the vent outlet.
28. A regulator for regulating the pressure and flow of a pressurized gas traveling between a gas inlet and first and second gas outlets of the regulator, comprising:
   a main line extending from the gas inlet;
   a first pressure regulator in fluid communication with the main line and configured to prevent the pressurized gas flowing downstream of the first pressure regulator from exceeding a first pressure;
   first and second supply lines extending from the main line downstream of the multiple position valve, the first and second supply lines being in fluid communication with the first and second gas outlets, respectively; and
   a multiple position valve in fluid communication with the main line, wherein the multiple position valve includes a plurality of positions corresponding to whether gas flows into only one of, or both of, the first and second supply lines and out of the first and second gas outlets, respectively.
29. The regulator of clause 28, wherein the first pressure is between approximately 25 psi and approximately 35 psi.
30. The regulator of clause 28, further comprising:
   a second pressure regulator in fluid communication with the first supply line and configured to prevent the gas flowing in the first supply line downstream of the second pressure regulator from exceeding a second pressure.
31. The regulator of clause 30, wherein the second pressure is between approximately 0 psi and approximately 30 psi.
32. The regulator of clause 31, wherein the second pressure is adjustable by a knob on a control interface module.
33. The regulator of clause 28, further comprising:
   a third pressure regulator in fluid communication with the second supply line and configured to prevent the gas flowing in the second supply line downstream of the third pressure control device from exceeding a third pressure;
   an auxiliary line extending from the second supply line upstream of the third pressure control device such that gas may flows in the auxiliary line at or below the first pressure;
   a pilot valve in fluid communication with the auxiliary line; and
   a control valve configured to move to an open position when the gas is transmitted to the pilot valve at or below the first pressure, wherein the control valve in the open position permits the gas to exit through the second gas outlet.
34. The regulator of clause 33, wherein the third pressure is between approximately 0 psi and approximately 15 psi.
35. The regulator of clause 28, further comprising:
   a switch valve in fluid communication with the main line and having open and closed positions, wherein gas is prevented from flowing downstream of the switch when the switch in the closed position and gas is permitted to flow downstream of the switch when the switch is in the open position.
36. The regulator of clause 28, further comprising:
   a vent line configured to be in fluid communication with a body cavity to thereby vent gas from the body cavity.
37. The regulator of clause 28, further comprising;
   a pressure relief valve in fluid communication with at least one of the main line, the first supply line, or the second supply line.
38. The regulator of clause 28, further comprising an adjustable flow control valve in at least one of the first or second supply lines.
39. A regulator for use with a gas-assisted applicator in an endoscopic or a laparoscopic procedure whereby the device is in fluid communication with a body cavity, comprising:
   an inlet configured to be fluidly communicated with a source of pressurized gas;
   an outlet in fluid communication with the inlet and configured to be coupled with a gas-assisted applicator; and
   a vent system including a vent line leading to a vent outlet, and a vacuum extraction system in fluid communication with the vent line, wherein the vent line is configured to be fluidly communicated with the body cavity such that gas within the body cavity may be drawn into the vent line and out of the vent outlet during operation of the vacuum extraction system.
40. The regulator of clause 39, further comprising:
   a line fluidly communicating the inlet and the vacuum extraction system, wherein gas flowing to the vacuum extraction system in the line causes the operation of the vacuum extraction system.
41. The regulator of clause 40, further comprising:
   at least one flow control device or pressure regulator upstream of the vacuum extraction system, in fluid communication with the line.
42. The regulator of clause 39, further comprising:
   a flow control device in fluid communication with the vent line.
43. The regulator of clause 42, wherein the flow control device is a flow control valve configured to control a flow rate of gas flowing into the vent line.
44. The regulator of clause 43, wherein the flow control valve is adjustable to thereby adjust the flow rate of gas flowing into the vent line.
45. The regulator of clause 43, wherein the flow control valve is preset such that the flow rate of gas flowing into the vent line is essentially not adjustable.
46. A system for use with a gas-assisted applicator in an endoscopic or a laparoscopic procedure whereby the device is in fluid communication with a body cavity, comprising:
   the regulator of clause 39; and
   a control interface module having a housing containing at least some components of the regulator, the module including a control interface for controlling at least one aspect of the regulator.
47. The system of clause 46, wherein:
   the regulator further comprises a flow control element in fluid communication with the vent line.
48. The system of clause 47, wherein the control interface module further comprises a vent inlet port on the outside of the housing and in fluid communication with the vent line of the regulator, wherein the flow control element is positioned upstream of the vent inlet port.
49. The system of clause 48, wherein the system further comprises:
   an external vent line having a first end and a second end and being configured to be fluidly communicated with the body cavity at the first end, and configured to be fluidly communicated with the vent inlet port at the second end to thereby fluidly communicate the vent line and the body cavity, wherein the flow control element is positioned between the external vent line and the vent inlet port.
50. The system of clause 46, wherein the flow control element is positioned within the housing.
51. The system of clause 46, further comprising:
   a gas-assisted applicator configured to be fluidly communicated with the outlet of the regulator.
52. A method of venting a body cavity during a gas-assisted laparoscopic or endoscopic procedure using a regulator comprising an inlet configured to be fluidly communicated with a source of pressurized gas, an outlet configured to be coupled with a gas-assisted endoscopic or laparoscopic device, the outlet in fluid communication with the inlet such that gas flowing into the inlet may flow to and be dispensed by the device into the body cavity, and a vent system including a vent line leading to a vent outlet, and a vacuum extraction system in fluid communication with the vent line, the method comprising:
   fluidly communicating the inlet to a source of pressurized gas;
   fluidly communicating the vent line to the body cavity; and
   operating the vacuum extraction system such that gas within the body cavity may be drawn through the vent line and out of the vent outlet.
53. The method of clause 52, wherein operating the vacuum extraction system further comprises:
   fluidly communicating the vacuum extraction system and the inlet, wherein gas flowing to the vacuum extraction system causes the vacuum extraction system to operate.
54. The method of clause 53, wherein the regulator further comprises a second line fluidly communicating the inlet and the vacuum extraction system and operating the vacuum extraction system further comprises:
   directing gas into the inlet, into the second line, and to the vacuum extraction system.
55. The method of clause 52, further comprising:
   adjusting a flow rate of the gas flowing out of the body cavity and into the vent line.

## Claims

1. A control interface module configured to control an internal regulator for regulating a pressure and flow of a pressurized gas flowing from a gas inlet to first and second gas outlets, comprising a housing having a gas inlet and first and second gas outlets, and a multiple position switch on the housing, the switch configured to control whether the gas is to permitted to flow from only one of, each of, or neither of the first and second outlets.

2. The control interface module of claim 1, wherein the switch includes first, second, and third positions, wherein each position of the switch corresponds to a different mode of operation, and wherein in a first mode, gas is permitted to flow out of only one of the first or second outlets, and in a second mode, gas is permitted to flow out of each of the first and second outlets.

3. The control interface module of claim 2, wherein in a third mode, gas is prevented from flowing from either of the first and second outlets.

4. The control interface module of any preceding claim, further comprising a knob configured to adjust pressure of the gas flowing from at first outlet.

5. A system for gas assisted delivery of at least one material, comprising the control interface module of any preceding claim and an applicator.

6. A method of performing a gas-assisted procedure using the control interface module of any one of claims 1 to 4, comprising:
fluidly communicating the inlet with a source of pressurized gas;
fluidly communicating at least one of the first or second outlets with an applicator; and
operating the switch to permit gas to flow out of at least one of the first or second outlets, corresponding to which of the first or second outlets is fluidly communicated with the applicator.

7. The method of claim 6, further comprising:
fluidly communicating only one of the first or second outlets with a applicator;
dispensing the one or more materials from the applicator onto a treatment site; and
wherein dispensing the at least one material from the applicator further comprises:
delivering the one or more materials into a body cavity endoscopically or laparoscopically.

8. A method of regulating a pressure and flow of a pressurized gas traveling between a gas inlet and a gas outlet of a regulator, comprising:
introducing pressurized gas into a first line of the regulator from an inlet of the regulator;
reducing the pressure of the gas to a first pressure using a first pressure regulator, thereby preventing gas flowing downstream of the first pressure regulator in the first line from exceeding the first pressure;
reducing the pressure of the gas flowing in the first line to a second pressure using a second pressure regulator, thereby preventing gas flowing downstream of the second pressure regulator in the first line from exceeding the second pressure;
transmitting the pressurized gas at or below the second pressure to a first pilot valve, thereby actuating a control valve to an open position;
transmitting the gas at or below the first pressure to a second pilot valve, thereby actuating a pneumatic valve to an open position; and
permitting the pressurized gas to flow downstream of the pneumatic valve in the first line and thereby flow out of the gas outlet when the pneumatic valve is in the open position.

9. The method of claim 8, wherein the regulator further comprises a second line extending from the first line to the second pilot valve, and the method further comprises:
transmitting the gas from the first line to the second line at or below the first pressure to the second pilot valve, thereby actuating the pneumatic valve to the open position.

10. A regulator for regulating the pressure and flow of a pressurized gas traveling between a gas inlet and first and second gas outlets of the regulator, comprising a main line extending from the gas inlet, a first pressure regulator in fluid communication with the main line and configured to prevent the pressurized gas flowing downstream of the first pressure regulator from exceeding a first pressure, first and second supply lines extending from the main line downstream of the multiple position valve, the first and second supply lines being in fluid communication with the first and second gas outlets, respectively, and a multiple position valve in fluid communication with the main line, wherein the multiple position valve includes a plurality of positions corresponding to whether gas flows into only one of, or both of, the first and second supply lines and out of the first and second gas outlets, respectively.

11. The regulator of claim 10, further comprising a second pressure regulator in fluid communication with the first supply line and configured to prevent the gas flowing in the first supply line downstream of the second pressure regulator from exceeding a second pressure.

12. The regulator of either claim 10 or claim 11, further comprising a third pressure regulator in fluid communication with the second supply line and configured to prevent the gas flowing in the second supply line downstream of the third pressure control device from exceeding a third pressure, an auxiliary line extending from the second supply line upstream of the third pressure control device such that gas may flows in the auxiliary line at or below the first pressure, a pilot valve in fluid communication with the auxiliary line, and a control valve configured to move to an open position when the gas is transmitted to the pilot valve at or below the first pressure, wherein the control valve in the open position permits the gas to exit through the second gas outlet.

13. The regulator of any one of claims 10 to 12, further comprising a switch valve in fluid communication with the main line and having open and closed positions, wherein gas is prevented from flowing downstream of the switch when the switch in the closed position and gas is permitted to flow downstream of the switch when the switch is in the open position.

14. The regulator of any one of claims 10 to 13, further comprising a vent line configured to be in fluid communication with a body cavity to thereby vent gas from the body cavity.

15. The regulator of any one of claims 10 to 14, further comprising a pressure relief valve in fluid communication with at least one of the main line, the first supply line, or the second supply line and an adjustable flow control valve in at least one of the first or second supply lines.

16. A system for use with a gas-assisted applicator in an endoscopic or a laparoscopic procedure whereby the device is in fluid communication with a body cavity, comprising a regulator having an inlet configured to be fluidly communicated with a source of pressurized gas, an outlet in fluid communication with the inlet and configured to be coupled with a gas-assisted applicator, and a vent system including a vent line leading to a vent outlet, and a vacuum extraction system in fluid communication with the vent line, wherein the vent line is configured to be fluidly communicated with the body cavity such that gas within the body cavity may be drawn into the vent line and out of the vent outlet during operation of the vacuum extraction system, and a control interface module having a housing containing at least some components of the regulator, the module including a control interface for controlling at least one aspect of the regulator.

17. The system of claim 16, wherein the regulator further comprises a flow control element in fluid communication with the vent line.

18. The system of claim 17, wherein the control interface module further comprises a vent inlet port on the outside of the housing and in fluid communication with the vent line of the regulator, wherein the flow control element is positioned upstream of the vent inlet port.

19. The system of claim 18, wherein the system further comprises an external vent line having a first end and a second end and being configured to be fluidly communicated with the body cavity at the first end, and configured to be fluidly communicated with the vent inlet port at the second end to thereby fluidly communicate the vent line and the body cavity, wherein the flow control element is positioned between the external vent line and the vent inlet port.
